# EUROPEAN PATENT APPLICATION

(11) **EP 1 184 032 A1**
(43) Date of publication of application: **06.03.2002**
(21) Application number: 00203030.2
(22) Date of filing: 31.08.2000
(51) Int. Cl.: A61K 9/16, C08B 37/00

(54) **Dextran hydrogels**

(71) Applicant: OctoPlus B.V., 2333 CA Leiden (NL)
(72) Inventor: Hennink, Wilhelmus Everhardus, 2743 CZ Waddinxveen (NL); Stenekes, Robert Jacob Herman, 9712 SR Groningen (NL)
(74) Representative: Prins, Adrianus Willem

(57) **Abstract**

The invention relates to a method for producing hydrogels, based on crystallization of dextran or derivatives thereof. These hydrogels find use in pharmaceutical, medical and biotechnological applications, e.g. as controlled release systems for the delivery of active ingredients in *in vivo* and *in vitro* applications. The hydrogels according to the present invention are priced by crystallization from an aqueous solution that is essentially free of organic solvents or crystallization enhancers.

## Description

The invention relates to a method for producing hydrogels, based on dextran or derivatives thereof. Hydrogels are hydrophilic polymeric networks, containing large amounts of water. These hydrogels find use in many different medical, veterinary, pharmaceutical and biotechnological applications.

The hydrogels of the present invention can, for example, be used as a matrix for the encapsulation of cells, which can be useful in tissue engineering. The hydrogels may also be used for topical administration, either *per se* or loaded with active ingredients, *e.g.* for the treatment of open wounds. Furthermore, the hydrogels can be used as systems for the delivery of active ingredients in *in vivo* and *in vitro* applications.

Active ingredients may be pharmaceutically interesting products, especially proteins, peptides, genetic material, genes, antigens and low molecular weight drugs; but also other types of active ingredients which are not of a pharmaceutical nature, such as flavors, nutrients or agrochemicals and so on, are envisaged. It is also possible to encapsulate colloidal systems, such as iscoms, liposomes, polyplexes, lipoplexes or nanoparticles.

Another very interesting application of the hydrogels of the present invention, which application takes advantage of the fact that dextrans can only be degraded in the body in the colon, is the use in oral route systems for targeted drug delivery in the colon.

Although different methods exist in the art for producing hydrogels, these methods used in practice have in common that they rely on organic solvents and/or chemicals in their preparation. Chemicals used are for example cross-linking agents or initiators. For many applications, such as the medical, veterinary, pharmaceutical and biotechnological applications mentioned above, the use of such chemicals poses limitations on the usefulness of the hydrogels that are obtained. For example, solvents like chloroform or dichloromethane required in some known hydrogel syntheses, have a detrimental effect on protein drugs. As a consequence, hydrogels based on these solvents cannot be used as delivery system for protein compounds.

Organic solvents may lead to structural changes in protein structure, especially in the secondary and tertiary structure. Such changes may lead to a denaturation of the protein drug. Since these structural changes normally lead to a loss in pharmacological activity and the occurrence of undesired side-effects, such changes are undesirable, as will be apparent. Moreover, the use of organic solvents is not desirable from an environmental point of view, either. Further, it is hardly possible to avoid that traces of organic solvents will remain in or on the products. Especially, when toxic solvents are used, such as the widely applied solvents chloroform and dichloromethane, this is a problem. Residual solvents might also affect the viability of cells in case of tissue engineering application.In order to obtain stable hydrogels *e.g.* for use in topical administration, often cross-linking agents are required in prior art methods. Many times, such cross-linking agents are not compatible with the envisaged application due to their toxicity.

Another problem is that it is often difficult to encapsulate proteins in polymeric matrices in a reproducible way. It is of the utmost importance that predictable and reproducible amounts of proteins or other encapsulated products to be used as drugs are released.

In summary, the known hydrogels pose problems with respect to toxicity with respect to the subject of application and/or stability of the active ingredients.

Dextran is a polysaccharide which consists essentially of α-1,6 linked D-glucopyranose residues with a low percentage of α-1,2, α-1,3 and α1,4 linked side chains. Dextran is soluble in water and highly compatible with living tissue. Therefore, dextran 40 000 (*viz*. dextran with a weight average molecular weight, Mw, of 40 000 Da) solutions (10 % w/v) are used as blood plasma substitute (see Aspinall, The polysaccharides. In: The polysaccharides. Academic Press, New York (1983)). However, a number of publications reported on limited stability of these dextran solutions (see: Cadwallader *et al*., J Am Pharm Assoc (Sci) **47** (1958) 894-895; Ewald *et al*., Military Med **129** (1964) 952-955; Hothorn, Pharmazie **30** (1975) 220-225; Hothorn, Pharmazie **30** (1975) 317-321; Aizawa *et al*., Bull Chem Soc Japan **49** (1976) 2061-2065; Veljkovic *et al*., Pharmazie **43** (1988) 840-842; Hirata *et al*., Biomaterials **20** (1999) 303-307).

Dextran based hydrogels have been under investigation as protein releasing matrix for a number of years (see *e.g.* Hennink *et al*., J Controlled Rel **39** (1996) 47-55; Van Dijk-Wolthuis *et al*., Macromolecules **30** (1997) 4639-4645; Franssen, *et al*. J Controlled Rel **44** (1997) 237-245; Franssen *et al*., J Controlled Rel **60** (1999) 211-221). Hydrogels can be obtained either by chemical or physical cross-linking of water-soluble polymers. Chemical cross-linking can be accomplished by reaction of the polymer with suitable bifunctional reagents like diisocyanates and epichlorohydrine or by derivatization of the polymer with reactive groups. An example of the latter is the coupling of methacryloyl groups to dextran, which can subsequently by polymerized by the addition of an initiator system, consisting of potassium peroxodisulfate (KPS) and *N,N,N',N'*-tetramethylethylenediamine (TEMED), resulting in a hydrogel (see: Van Dijk-Wolthuis *et al*., Macromolecules **28** (1995) 6317-6322; Van Dijk-Wolthuis *et al*., Macromolecules **30** (1997) 3411-3413; Van Dijk-Wolthuis *et al*., Polymer **38** (1997) 6235-6242). Physical cross-linking avoids the use of chemical reactions and can be based on *e.g.* ionic interactions or hydrophobic interactions. Another means to prepare hydrogels by physical cross-linking is based on stereocomplex formation of dextran-bound (L)- and (D)-lactic acid oligomers (see De Jong *et al*., Macromolecules **31** (1998) 6397-6402; De Jong *et al*. Macromolecules **33** (2000) 3680-3686).

As said, all of the above mentioned prior art methods for producing hydrogels *e*.*g*. for use as drug delivery systems, rely on organic solvents and/or chemicals for crosslinking or grafting the polymers which constitute the hydrogels. For this reason these prior art hydrogels are often less suitable for labile compounds, such as protein drugs. Also problems in administration may occur due to the toxicity of the compounds.

For these reasons, there is a need for hydrogels which do not rely on organic solvents and/or chemicals for chemical crosslinking or grafting the polymers which constitute the hydrogel.

It is an object of the present invention to provide a method for producing dextran hydrogels, which method does not rely on organic solvents or crosslinking chemicals which are required in prior art methods.

It has been found that this object can be met by producing a hydrogel of dextran or a derivative of dextran by allowing crystals to be formed from a dextran (or a derivative thereof) solution. Subsequently, the solution solidifies or gelates, by which the hydrogel is formed. Although crystallization of dextran is known in the art, these known methods for crystallization require pharmaceutically unacceptable compounds, such as methanol and/or depend on severe synthesis conditions (such as elevated temperature and/or pressure), to accomplish crystallization.

The present inventors surprisingly have found that dextran of a specific Mw crystallizes from aqueous solutions essentially spontaneously, viz. without requiring measures such as the addition of the above-mentioned pharmaceutically unacceptable crystallization enhancers or other compounds (although the presence of such compounds is sometimes desired for other reasons). Following the crystallization, a gel is formed. The dextran that can be used in accordance with the invention has an Mw of up to 20 000 Da, preferably the Mw is considerably less than 18 000, for instance in the range of 4000-10 000, more preferably in the range of 5000-7500, most preferably about 6000. When dextran of a Mw higher than 20 000 Da is used, *e.g.* a Mw of 50,000, the crystallization time becomes too long (more than 1 year).

Therefore, the present invention provides a method for producing a hydrogel of dextran or a derivative of dextran, which method comprises the steps of providing an aqueous solution of dextran or derivative thereof followed by crystallization of dextran or a derivative of dextran from said solution, by which said hydrogel is formed as a precipitate, characterized in that said solution does not comprise any organic solvents and that said crystallization may be carried out at room temperature and ambient (atmospheric) pressure. Preferably the method is carried out at about 25°C and atmospheric pressure.

When dextran crystallizes from solution, initially small crystals are formed, finally resulting in solidification or gelation of the solution. When these crystallites grow, water is trapped in the matrix formed by solid dextran material. Subsequently, a precipitate in the form of a gel is formed. Without wishing to be bound by any theory, it is believed that the gels of the present invention are formed by small crystalline domains which are formed from the solution. The crystalline domains act as "physical cross-links" between the amorphous domains and contribute considerably to the stability of the hydrogel.

It thus follows that, although the entire gel is not necessarily crystalline according to the present invention, it is essential that some crystallization has occurred in the formation of the hydrogels. As becomes clear from the above, the presence of crystalline domains gives rise to the formation of the gel. The present inventors have found that this crystallization takes place spontaneously with the specific dextran compounds, having the average molecular weight as specified herein.

It is to be understood that whenever mention is made of dextran in the present description and claims, also dextran derivates are to be understood. The dextran that is used for the present invention can be of a native, bacterial origin or it can be synthetic dextran, *e*.*g*. obtained by polymerization according to the method of Schuerch and Uryu ("Macromolecular Syntheses", John Wiley and Sons, New York **4** (1972) 151). Usually the dextran is slightly branched with *e*.*g*. 5 % of the material in the branches.

Dextran derivates may be obtained by substituting dextran with a suitable group, *e.g.* a methacrylate group. This substitution is carried out to a certain degree of substitution, DS, which should not be too high, since this would hamper the crystallization and subsequent gelation of the dextran. It was found that for dextran with a number average Mn of 4300 Da (corresponding to a degree of polymerization of 26-27), one branching per dextran chain (on the average) does not disrupt the crystallization and subsequent precipitation. This was demonstrated by ¹H-NMR studies where it was shown that precipitates that are formed in accordance with the present invention, may contain about 3.5 % branching, which corresponds to one branching point per 28 glucopyranose units.

The solutions from which the dextran crystallize, and subsequently gelate, are formed by dissolving the dextran using suitable equipment, such as a vortex mixer. This may be carried out at ambient conditions. After dissolving the dextran, the crystallization takes place essentially spontaneously, *i.e.* without further measures being taken, at ambient conditions. Crystallization of the dextran may be helped *e.g.* by stirring, such as by using a laboratory magnetic stirrer.

Surprisingly, it was furthermore found that the crystallization rate lowered at 4°C. This finding can be used to store solutions at 4°C, while these solutions only start to form gels when they are removed from the cooled storage and are brought to higher temperature, such as room temperature.

Apart from the Mw, the crystallization is influenced by the concentration of the dextran solution. The starting solutions may have a concentration of 15-70 wt.%, preferably 40-70 wt.% dextran. When the concentration is lower than 15 wt.%, the crystallization rate will be too slow. On the other hand, when the concentration becomes higher than 70 wt.%, the dextran will not dissolve completely.

Another distinct advantage of the present invention is the relative simplicity at which the process may be carried out. The crystallization process can easily be stopped by diluting with water. After this the supernatant liquid may be separated from the precipitate. The hydrogel may subsequently be dried, *e.g.* by freeze-drying. The freeze dried product can be stored. Upon contact with water it can be converted in a gel again for further use.

By stopping the crystallization/precipitation process at a desired point in time, microspheres can be produced. The diameter thereof can be controlled from less than 1 µm to several tens of µm with a very narrow size distribution.

The crystallization/precipitation rate may be increased by addition of salts, such as buffer salts, to the solution. Typical salts which are employed for this purpose are phosphate buffer, KCl, HEPES and/or NaCl.

The gels of the present invention may be used *per se*, *e*.*g*. for topical administration. Alternatively, they may be loaded with a drug or other active ingredient, which can be released upon application of the gel. In order to accomplish this, the active ingredient may be mixed in the initial dextran solution. As a result the active ingredient will be incorporated mainly in the amorphous dextran phase. Alternatively, the gel may be loaded with an active ingredient after the gel has been formed.

Apart from as a controlled release agent, the crystalline dextran or dextran derivative of the invention may be used in the preparation of hydrogels which can be used as a matrix for growing and maintaining cells. Also a composition for topical administration, as mentioned above, can be made in this way, *e*.*g*. in the form of an ointment. These composition can *e.g.* be used for the treatment of open wounds, burns, *etc.*

As mentioned herein-above, the precipitate obtained from crystalline dextran or derivative thereof, may be used in the preparation of a hydrogel that can be used as targeted release of compounds in the colon. Therefore, the invention also relates to the use of crystalline dextran (or derivative thereof) in the preparation of a medicament for the treatment of a disorder in, of or associated with, the colon.

### Description of the drawings

In Figure 1 the absorbance at 450 nm of a 50 % dextran 6000 solution as function of time is given.

Figure 2 shows a particle size distribution plot of microspheres formed from a 50 % w/w dextran 6000 solution.

In Figure 3 a SEM-picture of microspheres formed from a 50 % w/w dextran 6000 solution is shown.

Figure 4 shows the kinetics of the precipitation process in 50 (on standing (■) and stirring (▲)) and 60 % (○) dextran 6000 solutions in doubly distilled water (expressed as weight percentage precipitates of total amount of dextran 6000 added).

Figure 5 shows the storage modulus (G') at 20°C as function of time for (A) 60, (B) 50 and (C) 40 % dextran 6000 solutions.

Figure 6 shows IR-spectra of (A) non-precipitated dextran and (B) precipitates.

Figure 7 shows DSC-thermograms of (A) non-precipitated dextran and (B) precipitates. The non-precipitated dextran has a glass transition at 204.5°C and the precipitates have a glass transition at 220.7°C (Δ*H* = 17.8 J/g). The broad peaks between 30 and 150°C are due to residual water (*ΔH =* 140 J/g; see also Figure 8).

Figure 8 shows TGA-thermograms of non-precipitated dextran (- - -) and precipitates (_).

### Example 1

### Formation of precipitates

30, 40, 50 and 60 % wt. dextran 6000 solutions were made by transferring 1.5, 2.0, 2.5 and 3.0 g dextran 6000 (purchased from Fluka, Buchs, Switzerland), respectively into a scintillation vial and subsequently adding 3.5, 3.0, 2.5 and 2.0 g reversed osmosis water (*i*.*e*. doubly distilled water), respectively. To accelerate dissolution, the mixtures were vortexed and subsequently put on a roller bench, on which the vials are rotated around their longest axis at a slow speed (about 40 rpm). When the dextran was completely dissolved, the vial was taken off the roller bench.

Precipitates formed spontaneously on standing or by stirring of the solution by a laboratory magnetic stirrer. At the desired time point, the precipitation process was stopped by dilution with water.

Depending on the precipitation time, microspheres or gels could be obtained. Subsequently, the mixture was transferred to a 50 ml conical tube and the precipitates were collected by centrifugation (10 min, 3578 × g) and washed three times with water. The supernatants were collected as well. Both the precipitates and supernatants (containing non-precipitated dextran) were freeze-dried prior to further analysis and the yield of the precipitates was determined.

### Results

**Formation of precipitates** Addition of reversed osmosis water to dextran 6000 (final concentration of 30-60 % w/w) results, after a short dissolution time of about 15 minutes, in a clear solution. Under ambient conditions, in time, considerable amounts of precipitates were formed with yields up to 70 ± 6 % (n = 3) of the dextran initially added (after 2 weeks). Precipitation was not observed for higher molecular weight dextrans (40 000 and 220 000) in the time-frame studied.

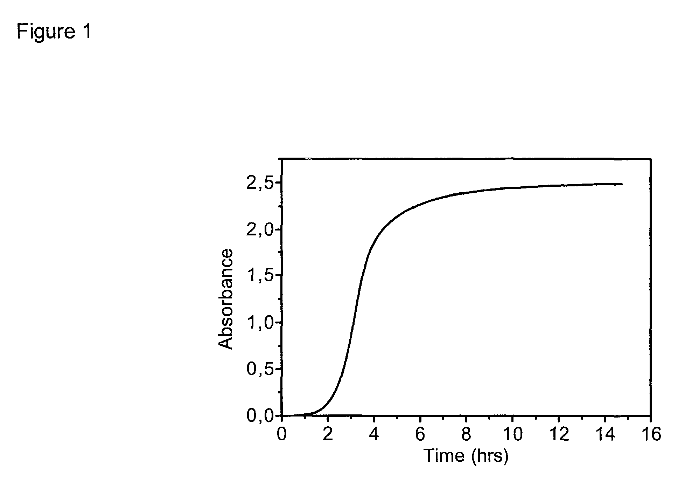

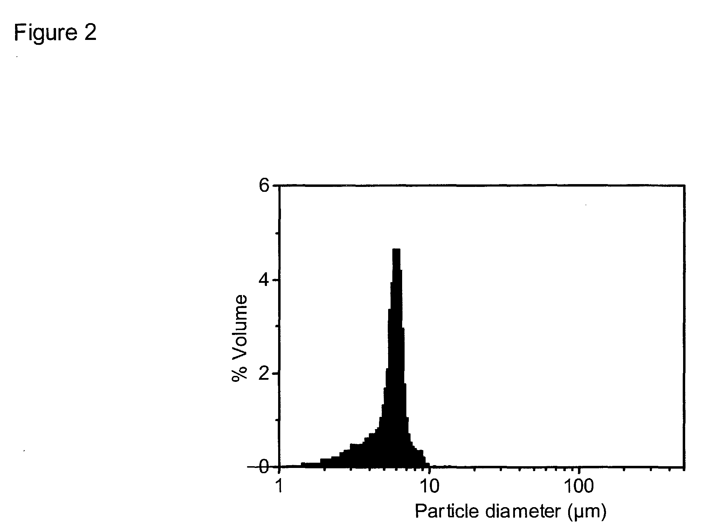

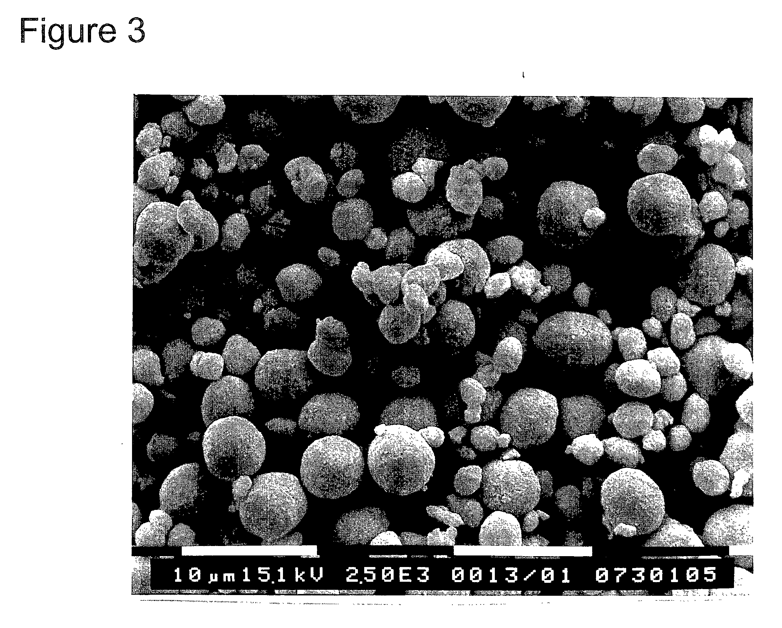

A few hours after dissolution, the dextran 6000 solution becomes turbid (Figure 1) and the precipitation process proceeded until the solution was completely gelled. When the process of precipitate formation (under stirring) is stopped before gelation (by diluting the turbid solution in water), the precipitates were spherically shaped and had a narrow size distribution as shown in Figures 2 and 3, respectively.

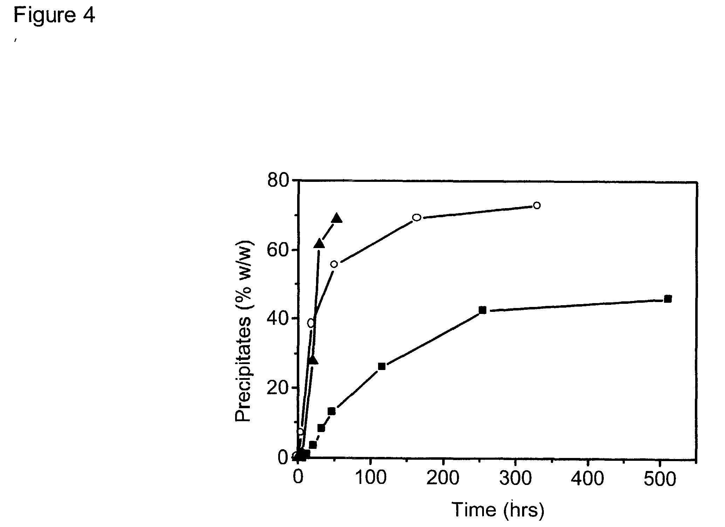

Precipitation was faster when more concentrated dextran solutions were used, when the solution was stirred (Figure 4 and Table I) and in the presence of salts, such as 10 mM phosphate buffer, 0.22 M KCl or HEPES + 0.8 wt.% NaCl (results not shown).

**Table I**

| Yield of precipitates after two weeks for dextran 6000 solutions with different concentrations. | |
|---|---|
| Concentration (% w/w) | Yield (%) |
| 20 | 0 |
| 30 | 0.1 |
| 40 | 4.2 |
| 50 | 49.7 |
| 60 | 73.0 |

From the results in Table I it also follows that crystallization may be stopped by dilution; at low dextran concentrations there is no crystallization.

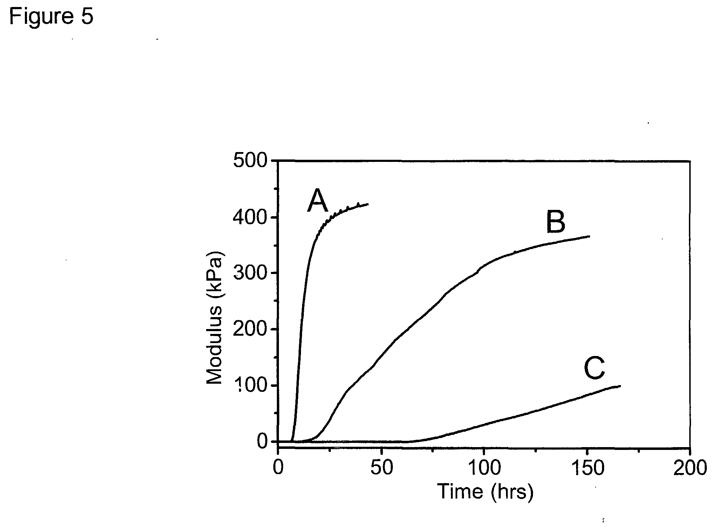

**Rheology measurements** The gel formation of dextran 6000 solutions was also studied by rheology measurements. In Figure 5, the storage modulus G' is plotted against time for three solutions. After an initial lag-time, the G' increased indicating that gels were formed. The lag-time decreased with increasing dextran 6000 concentration. Furthermore, the plateau value for G' is higher for more concentrated solutions. This demonstrates again that in a more concentrated dextran 6000 solution, precipitation occurs more rapidly. The precipitates did not dissolve in water at room temperature, whereas low concentrations (15 mg/ml) did dissolve upon heating (60 minutes, 100°C) or in DMSO. Once the precipitates were dissolved by heating, the solution remained clear upon cooling down, because the dextran is diluted to low concentrations (1.5 % w/w) at which no precipitation occurs.

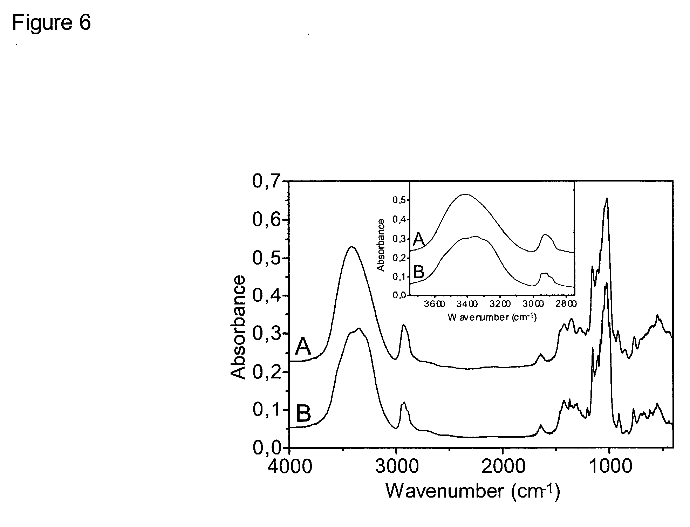

**Infra-Red spectroscopy** IR spectroscopy showed that the precipitates and the soluble dextran fraction were structurally related (Figure 6). However, the precipitates gave sharper peaks than the non-precipitated dextran, e.g. for the CH/CH₂ vibrations at 2900 cm⁻¹ and the OH vibrations at 3400 cm⁻¹ (see insert). This means that in the precipitates the atoms have restricted mobility, which means that the precipitate is crystalline.

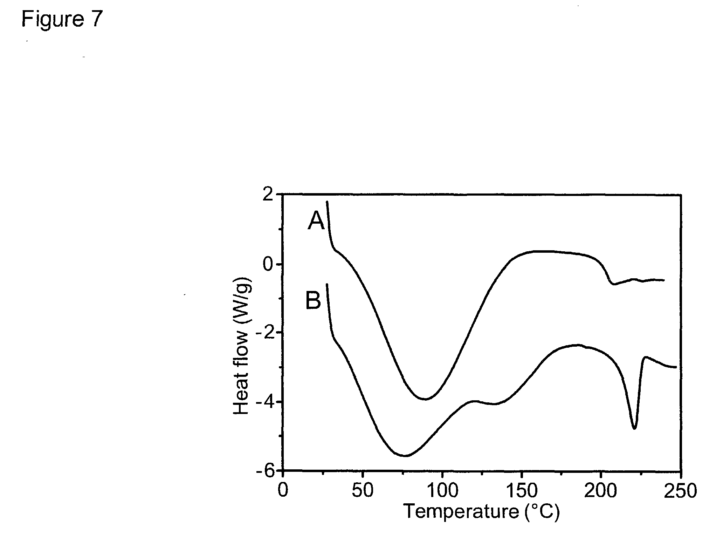

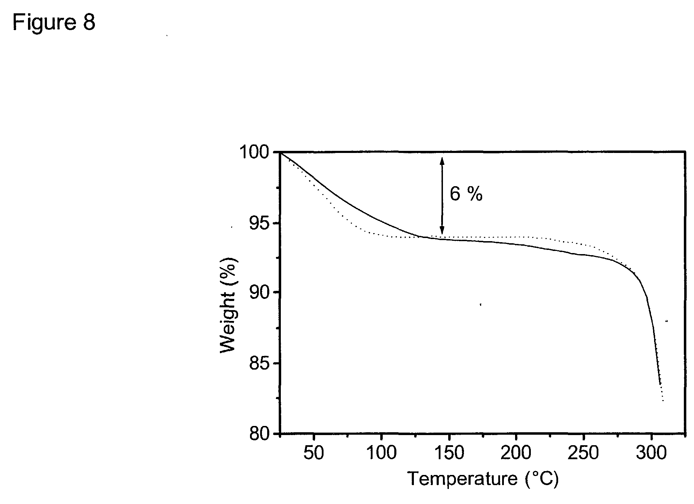

**(Modulated) differential scanning calorimetry** (M)DSC measurements also show that the precipitates were crystalline. In Figure 7, the DSC thermograms of both precipitates and the non-precipitated dextran are presented. For both samples, the broad endothermal peak between 30 and 150°C is due to the evaporation of residual water. The enthalpy change of this peak is 140 J/g, which corresponds to 6 % water (the evaporation enthalpy of pure water is 2260 J/g). This is in agreement with the residual water content determined by thermogravimetric analysis (TGA, figure 8). Furthermore, Figure 7 shows, for the non-precipitated dextran, a transition at 204.5 ± 0.6 °C, similar to untreated dextran (observed in the reversing heat flow; not shown). However, the precipitates have a melting peak at 220.7 ± 0.1 °C with a *ΔH* of 17.8 ± 1.2 J/g. This indicates that the precipitates are at least partially crystalline.

¹H-NMR spectroscopy demonstrated that precipitated dextran had a slightly lower degree of branching (3.5 ± 0.2 % (n=3)) than the non-precipitated dextran (5.4 ± 0.5 % (n=3)). The 3.5 % branching for the precipitates, corresponds to 1 branching point per 28 glucopyranose units.

Gel permeation chromatography demonstrated that the dextran of the precipitates had a slightly higher molecular weight than the non-precipitated dextran (Table II). The number average molecular weight of the precipitates was 4500 g/mol, which corresponds to an average degree of polymerization of 4500/162 = 28. Combined with NMR-data, this means that the precipitates on average contain one branching point per chain.

**Table II**

| Molecular weights and polydispersity (D) of untreated, non-precipitated and precipitated dextran. | | | |
|---|---|---|---|
| | M_{w} (g/mol) | Mₙ (g/mol) | D |
| Untreated | 7100 ± 200 | 4300 ± 100 | 1.65 |
| Non-precipitated | 6800 ± 200 | 4200 ± 100 | 1.62 |
| Precipitated | 7200 ± 200 | 4500 ± 100 | 1.60 |

### Example 2

### Re-use of soluble fraction

Freeze-drying of the soluble fraction of dextran and subsequent preparation of a 50 % w/w solution in water, again resulted in precipitate formation, indicating that it is not a specific fraction of dextran that precipitated.

### Example 3

### Release of proteins from Dex 6000 gels

The release of three different types of protein (IgG, BSA and lysozyme) from a gel prepared from dextran with a Mw of 6000 Da ("dextran 6000") was studied.

The protein loaded gels were prepared by making three different sets of solutions, comprising 50 and 60 wt.% dextran 6000. This was done by adding to solid dextran a protein phosphate buffer solution comprising one of the three proteins. In this way six vials were filled with a gel comprising 5 mg protein per gram of resultant gel. Crystallization/precipitation was carried out as described in Example 1. After crystallization, each vial contained 12.5 g of protein loaded gel.

In order to study the protein release the loaded gels were transferred to a tube and 10 ml 100 mM phosphate buffer was added. The tubes were put on a roller bench, similar to the one described in Example 1. From each tube 2.5 ml samples were drawn at predetermined intervals. After drawing a sample, the tubes were refilled up to 10 ml buffer solution. Of these samples 25 µl (for lysozyme) or 20 µl (for BSA and IgG) was transferred to an Eppendorf vial. Subsequently, 100 mM phosphate buffer was added to 1.0 ml. Five minutes prior to a measurement, 250 µl Biorad™ reagens was added, after which the extinction at 595.0 nm was measure on a Perking Elmer Lambda 2, operated with PECSS software. From this measurement the concentration of protein could be derived. The protein release data from the 50 % and 60 % dextran 6000 gels is given in Table 2.

**Table 2.**

| Protein release data from dextran hydrogels | | | | |
|---|---|---|---|---|
| | 50 wt.% dextran gel | | 60 wt.% dextran gel | |
| protein | t_{50%}/[h]¹⁾ | t_{90%}/[h] ²⁾ | t_{50%}/[h] ¹⁾ | t_{90%}/[h] ²⁾ |
| IgG | 5 | 80 | 4 | 40 |
| BSA | 5 | 70 | 3 | 20 |
| lysozyme | 5 | 60 | 4 | 24 |

| | | | | |
|---|---|---|---|---|
| ¹⁾ Time at which 50 wt.% of protein is released from the hydrogel. | | | | |
| ²⁾ Time at which 90 wt.% of protein is released from the hydrogel. | | | | |

These results illustrate that the gels of the present invention can be used for sustained release of proteins and is thus a suitable controlled release agent.

### Example 4

### Crystallization of Methcrylate substituted dextran

A 50 wt.% solution of dexMA (dextran substituted with methacrylate, degree of substitution 5.5; *viz*. 5.5 groups per 100 glucose units) was prepared as described in Van Dijk-Wolthuis *et al*. (Macromolecules **28** (1995) 6317-6322; and Macromolecules **30** (1997) 3411-3413). Initially, a clear solution was obtained. Stirring at ambient conditions resulted in the formation of a gel after 3 days.

This Example demonstrates that derivatized dextran can be used as well for a method which involves crystallization, followed by precipitation, to form hydrogels in accordance with the present invention.

## Claims

1. Method for producing a hydrogel of dextran or a derivative of dextran, comprising the steps of providing an aqueous solution of dextran or derivative thereof with a weight average molecular weight (Mw) of 2000 - 18 000 Da, followed by crystallization of dextran or a derivative of dextran from said solution, forming said hydrogel as a precipitate.

2. Method according to claim 1, wherein Mw is about 6000 Da.

3. Method according to claim 1 or 2, which is followed by a step wherein the precipitation is stopped by dilution of the solution with water.

4. Method according to any of the previous claims, which is followed by a step wherein said precipitate is separated from the solution, optionally followed by a step wherein said precipitate is dried.

5. Method according to any of the previous claims, wherein said aqueous solution is essentially free of organic solvents.

6. Method according to any of the previous claims, wherein the concentration of dextran or dextran derivative in said solution is 15-70 wt.%, preferably 40-60 wt.%.

7. Method for producing a hydrogel of dextran or a derivative of dextran, comprising the steps of providing an aqueous solution of dextran or derivative thereof, followed by crystallization of dextran or a derivative of dextran from said solution, forming said hydrogel as a precipitate, **characterized in that** said solution does not comprise any organic solvents and that said crystallization may be carried out at room temperature and ambient pressure.

8. Method according to claim 7, which is carried out at about 25°C and atmospheric pressure.

9. Method according to any of the previous claims, wherein an active ingredient is present in said solution, whereby a gel loaded with said active ingredient is obtained.

10. Method according to claim 9, wherein said active ingredient is selected from cells, proteins, genetic material, peptide, colloidal drug carriers, low molecular weight drugs, and flavors.

11. Product comprising crystalline dextran or dextran derivative, wherein the dextran or dextran derivative has a weight average molecular weight (Mw) of 2000 - 18 000 Da.

12. Product of claim 11 wherein the dextran or dextran derivative has a Mw of about 6000 Da.

13. Use of crystalline dextran or dextran derivative in the preparation of a controlled release agent; a matrix for growing and maintaining cells; or a composition for topical administration.

14. Use of crystalline dextran in the preparation of a medicament for the treatment of a disorder of, in, or associated with, the colon.
